# EUROPEAN PATENT SPECIFICATION

(11) **EP 3 315 510 B1**
(45) Date of publication and mention of the grant of the patent: **19.02.2025**
(21) Application number: 15893723.5
(22) Date of filing: 03.06.2015
(51) Int. Cl.: C07K 14/54, C07K 14/52, A61P 35/00, A61K 38/20, A61K 38/19

(54) **MODIFIED CHEMOKINE PEPTIDE**
MODIFIZIERTES CHEMOKINPEPTID
PEPTIDE DE CHIMIOKINE MODIFIÉ

(43) Date of publication of application: 02.05.2018
(73) Proprietor: Rise Biopharmaceuticals, Inc. (Beijing), Beijing City (CN)
(72) Inventor: CHENG, Hsi-Tsung, Tainan City 701 (TW); CHENG, Jya-Wei, Tainan City 701 (TW); YU, Hui-Yuan, Tainan City 701 (TW)
(74) Representative: Glück Kritzenberger Patentanwälte PartGmbB
(86) International application number: PCT/CN2015/080725
(87) International publication number: WO 2016/192067

(56) References cited:
- EP-A1- 0 616 615
- CN-A- 101 200 502
- CN-A- 102 596 227
- US-A1- 2010 029 562
- US-A1- 2013 109 614
- CHENG H T ET AL: "A new protocol for high-yield purification of recombinant human CXCL8"("3"-"7"2")K11R/G31P expressed in Escherichia coli", PROTEIN EXPRESSION AND PURIFICATION, ACADEMIC PRESS, SAN DIEGO, CA, vol. 61, no. 1, 1 September 2008 (2008-09-01), pages 65 - 72, XP023182968, ISSN: 1046-5928, [retrieved on 20080501], DOI: 10.1016/J.PEP.2008.04.007
- LE, Y.Y. ET AL.: "Chemokines and Chemokine Receptors: their Maniold Roles in Homeostasis and Disease", CELLULAR & MOLECULAR IMMUNOLOGY, vol. 1, no. 2, 30 April 2004 (2004-04-30), XP055331932

## Description

### FIELD OF THE INVENTION

The present invention relates to a modified chemokine peptide capable of being a therapeutic antagonist. In particular, the present invention relates to a modified chemokine for treating cancer and inhibiting tumor growth.

### BACKGROUND OF THE INVENTION

Chemokines are a group of inducible, secretory, structurally and related small molecules (approximately 8 to 14 kD). Chemokine is divided into four subfamilies, such as CXC, CC, CX3C and C. Chemokines are also grouped into two main functional subfamilies: "homeostatic chemokines" and "inflammatory chemokines.

Chemokine usually has three β-sheets in its structure, and has a α-helix at C terminal and 4-conserved cysteines at N-terminus. Chemokines have been categorized into four subfamilies (CXC, CC, CX3C and XC) based on the sequence containing the first two cysteines (Cys) at N-terminus. Among the four types, CXC and CC are the two major subfamilies while XC and CX3C are minor subfamilies. Reaction happens after chemokine on the cell conjugated with chemokine receptor. Chemokine receptor includes seven transmembrane G protein binding receptors, which respectively are nominated as CXCR, CCR, CXR and CX₃CR according to the types of ligands on the binding target. Chemokine receptors are further arranged according to the number, such as CXCR1, CXCR2, CXCR4 and so on. However, not only one chemokine receptor is represented on the target cells, the binding target of the infiltrated inflammation cells is not specifically to one chemokine receptor, and then some chemokine receptors of different cells must be expressed under certain stimulation and induction.

For instance, ELR-CXC chemokine with glutamate (E)-leucine (L)-Arginine (R) characteristic sequence (ELR characteristic sequence) is referred to a protein having the amino acid sequence of ELR-CXC characteristic at N-terminus, and X would be the amino acid having polarity with or without charge, or X is absent. ELR-CXC chemokine can regulate the expression of carcinogen, IL-8 and neutrophil-activating protein-2 (NAP-2). Their receptors are CXCR1 and CXCR2, and they mainly target to neutrophils. ELR-CXC chemokine can promote the accumulation and activation of neutrophils. Thus, this type of ELR-CXC chemokine plays an important role in the generation of the board-ranged acute and chronic inflammation diseases. These inflammations include psoriasis and rheumatoid arthritis.

Additionally, ELR-CXC chemokine is associated with angiogenesis accompanied upon tumor development, and its inductive mechanism is the activation generated by conjugating this type of chemokine, especially referring to IL-8 (CXCL8), with CXCR1 and CXCR2 on the endothelial cells (ECs). At present, it is proved that many different types of tumors are able to secret ELR-CXC chemokines, and the tumors overexpressing these chemokines would be associated with poor prognosis.

Anti-conjugation of CXCR1 or CXCR2 with ELR-CXC chemokine is a practicable strategy, so that the abnormal signal transmission induced by the activation of CXCR1 or/and CXCR2 receptor is inhibited, so that the associated diseases resulted from the activation of cells with two types of receptors are treated. Accordingly, scientists are endeavoring in finding and preparing receptor protein analogs for inhibiting CXC chemokines.

It is therefore attempted by the applicant to deal with the above situation encountered in the prior art.

### SUMMARY OF THE INVENTION

Cancer is the most popular disease cause of death in developed countries. If cancer is diagnosed at an early stage, it is more likely to be treated successfully. Although there has been considerable progress in the diagnosis and treatment of cancer, these drugs are either causing serious side effects or ineffective. Therefore, a novel method or a novel composition for treating cancer or preventing cancer is needed.

In order to solve the above-mentioned problems, according to one embodiment of the present invention, there is provided a modified chemokine for inhibiting tumor growth and treating cancer according to claim 1. Preferred embodiments of the modified chemokine are represented by the dependent claims 2 to 4.

The present invention provides a modified chemokine peptide comprising a peptide sequence. The N-terminus of the peptide sequence comprises two characteristic sequences. One characteristic sequence is a "(a) Glutamate (E)-Leucine (L)-Arginine (R) sequence" which is situated at the N-terminus of the modified chemokine peptide. Another characteristic sequence is a "(b) Proline (P)-Alanine (A)-Serine (S)-Glutamine (Q)-Phenylalanine (F) sequence" which is neighbored to the upstream of the third cysteine (C) counted from N-terminus of a chemokine peptide. The modified chemokine peptide comprise (a) characteristic sequence, (b) characteristic sequence, and a modified position. The above-mentioned modified position is situated at the 17^{th}, 12^{th}, or 13^{th} position counted from the N-terminus of the modified chemokine peptide.

In one embodiment, the phenylalanine (F) residue at position 17^{th} is substituted with leucine (L).

The un-modified precursor of the modified chemokine peptide is originated from a source chemokine. Zero to two amino acid residue is located between the first two cysteines at N-terminus of the source chemokine, and the amino acid residue has polarity with or without charge, when the number of the amino acid residue is one to two.

According to the present invention, the source chemokine peptide is selected from the group consisting of SEQ ID NO:1 and SEQ ID NO: 2. Source chemokine peptides according to SEQ ID NO:2, SEQ ID NO:3, SEQ ID NO:4, SEQ ID NO:5, SEQ ID NO:6, SEQ ID NO: 7, SEQ ID NO: 8, or SEQ ID NO: 9 as disclosed herein are not according to the present invention.

In one embodiment, the modified chemokine peptide is selected from SEQ ID NO:10 or SEQ ID NO: 12.

The present invention also provides a pharmaceutical composition, comprising the modified chemokine peptide of the present invention and a pharmaceutically acceptable excipient.

In one embodiment, the modified chemokine peptide is suitable for treating cancer or inhibiting tumor growth.

The present invention further provides a pharmaceutical composition for treating cancer or inhibiting tumor growth, comprising a therapeutically effective amount of a modified chemokine peptide of the present invention and a pharmaceutically acceptable excipient. Preferred embodiments are represented by dependent claims 8 and 9.

In one embodiment, the cancer comprises prostate cancer, breast cancer, uterine cancer, leukemia, ovarian cancer, endometrial cancer, cervical cancer, colorectal cancer, testicular cancer, lymphoma, rhabdomyosarcoma, neuroblastoma, pancreatic cancer, lung cancer, brain tumor, skin cancer, stomach cancer, oral cancer, liver cancer, laryngeal cancer, gallbladder cancer, thyroid cancer, liver cancer, kidney cancer, or nasopharyngeal carcinoma.

### BRIEF DESCRIPTION OF THE DRAWINGS

Fig. 1 illustrates the comparison of sequence alignment to the source chemokine peptide sequences (SEQ ID NO: 1 to 9), i.e. the amino acid sequences of ELR-CXC chemokines with high affinity to CXCR1 or/and CXCR2.
Fig. 2 illustrates the comparison of sequence alignment to the amino acid sequences of the modified chemokine peptides (SEQ ID NO: 10 to SEQ ID NO: 12) in the invention.
Fig. 3 illustrates that the largest amount of cells was migrated in the present of CXCL8.
Fig. 4 illustrates the number of migrated cells. Black bar represents chemotaxi are triggered by CXCL8. Gray bar represents treatment with ELR-CXC chemokine and CXCL8-IP10 both. White bar represents treating with ELR-CXC chemokine and CXCL8-17LIP10 both.
Fig.5 illustrates the tumor volume of treatment group (IL8-17LIP10) and placebo group (saline).
Fig.6 illustrates the tumor weight of treatment group (IL8-17LIP10) and placebo group (saline).
Fig.7 illustrates the microvessel density of treatment group (IL8-17LIP10) and placebo group (saline).
Fig. 8 illustrates the tumor volume after injection of CXCL8-IP10 or saline. Group A was injected 500 µg/kg of CXCL8-IP10 four times weekly. Group B was injected 500 µg/kg of CXCL8-IP10 twice weekly. Group C was injected 250 µg/kg of CXCL8-IP10 twice weekly. Group D was subcutaneously injected 100 µl of saline every day.
Fig. 9 show the images for external appearance of tumor tissues obtained from mice lung. Mice were sacrificed on day 24 after administration with 500 µg/kg i.p. of CXCL8-IP10 four times weekly (Fig. 9A) or saline (Fig. 9B) to obtain the lung tissue. The white nude indicated by arrow was nidus of the cancer which has spread into lung.
Fig. 10 illustrates the expression level of CXCR1 and CXCL8 in cancer cells.
Fig. 11 illustrates the efficacy of the antagonism of the invention by chemotaxis.

### DETAILED DESCRIPTION OF THE INVENTION

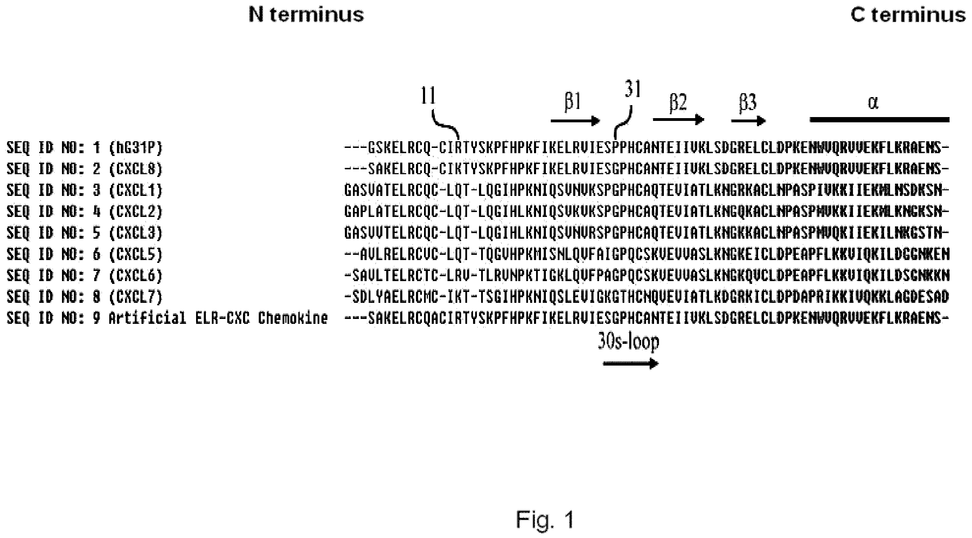

The present invention provides a novel chemokine. The novel chemokine of the present invention is derived from ELR-CXC chemokine or a substance having high binding affinity to CXCR1 or CXCR2, namely CXCL8 (SEQ ID NO: 2), and hG31P (SEQ ID NO: 1). In the present invention, modification is made in accordance with this type of chemokine in this invention, and it mainly depends on a PASQF characteristic sequence substituted the original 30s-loop region (Fig.1). This PASQF originally exists in CXCL 10 chemokine, which is a non-ELR-CXC chemokine.

The modified chemokine of the present invention comprises (a) "-N'-Glutamate (E)-Leucine (L)-Arginine (R)" characteristic sequence which is situated at the N-terminus of the modified chemokine peptide, and (b) a "-N'-Proline (P)-Alanine (A)-Serine (S)-Glutamine (Q)-Phenylalanine (F)-Cys³" characteristic sequence which is neighbored to the upstream of the third cysteine (C) counted from N-terminus of the chemokine peptide. It shall be noted that the modified chemokine of the present invention further comprises a mutation (modification) at the 17^{th} position counted from the N-terminus of the modified chemokine peptide.

The original amino acid residue at position 17 is phenylalanine (F), and the 17^{th} amino acid residue is substituted to non-phenylalanine (F) amino acid by a single amino acid mutation. The amino acid residue at position 17 is substituted to leucine (L), isoleucine (I), or valine (V), , preferably leucine (L). Compared with the source chemokine peptide without any amino acid substitution, the modified chemokines have a higher affinity and could effectively inhibit tumor growth after an amino acid residue substituted at position 17.

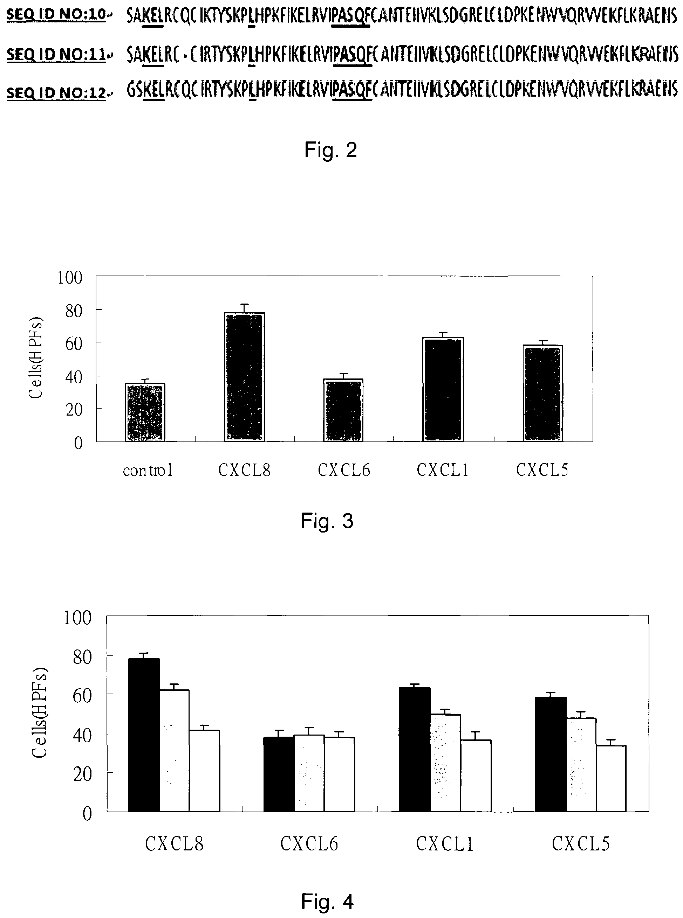

Optionally, in an exemplary embodiment of the present invention, the modified chemokines of the present invention include, but are not limited to, SEQ ID NO: 10 and/or SEQ ID NO: 12 (Fig. 2).

For example, SEQ ID NO: 10 not only includes a N-terminal sequence of ELR-CQC to satisfy the rule of amino acid characteristic sequence of ELR-CXnX, but also has an oligopeptide sequence, Pro-Ala-Ser-Gln-Phe (PASQF). PASQF is a modified sequence substituted for the upstream of the third cysteine (10192) counted from N-terminus, and this third cysteine closely neighbors to phenoalanine of PASQF oligopeptide sequence. More importantly, the amino acid residue at 17^{th} position (the first modified position) is leucine, not phenylalanine.

According to sequences shown in Fig.2, SEQ ID NO: 10, SEQ ID NO: 11, and SEQ ID NO: 12 of the ELR-CXC chemokine analogs have a PASQF modified sequence, which is neighbored to the upstream of the third cysteine (C) counted from N-terminus of a chemokine peptide. Additionally, the amino acid residue at position 17 of the chemokine of the present invention is mutated to leucine.

The modified chemokine peptides of the present invention can treat diseases associated with angiogenesis. The angiogenesis associated diseases include, but are not limited to, inflammatory disorders, chronic articular rheumatism and psoriasis, disorders associated with inappropriate or inopportune invasion of vessels such as diabetic retinopathy, neovascular glaucoma, restenosis, and cell proliferation disorder/disease, such as neoplasm and cancer associated disorders (e.g., prostate cancer, breast cancer, uterine cancer, leukemia, ovarian cancer, endometrial cancer, cervical cancer, colorectal cancer, testicular cancer, lymphoma, rhabdomyosarcoma, neuroblastoma, pancreatic cancer, lung cancer, brain tumor, skin cancer, stomach cancer, oral cancer, liver cancer, laryngeal cancer, gallbladder cancer, thyroid cancer, liver cancer, kidney cancer, or nasopharyngeal carcinoma).

The modified chemokine peptides of the present invention can be administered orally, buccally, parenterally, by inhalation spray, rectally, intradermally, transdermally, or topically in dosage unit formulations containing conventional nontoxic pharmaceutically acceptable carriers, adjuvants, and vehicles as desired.

The modified chemokine peptides of the present invention can be administered in a single dose, in multiple doses throughout a 24-hour period, or by continuous infusion. When administered by continuous infusion, the compounds can be supplied by methods well known in the art, such as, but not limited to, intravenous gravity drip, intravenous infusion pump, implantable infusion pump, or any topical routes. Length of treatment will vary depending on many factors, for example, the duration and severity of the angiogenesis condition. Treatment of the subject with the modified chemokine of the present invention alone or in combination with other agents may last until the angiogenesis disappears, or treatment will continue for the life of the subject.

In another embodiment, the present invention provides a pharmaceutical composition for treating cancer and inhibiting tumor. The pharmaceutical composition comprises an effective amount of a modified chemokine of the present invention or analogs thereof and a pharmaceutically acceptable carrier. The pharmaceutically acceptable carrier could include solvent, dispersants, coating, antibacterial/antifungal agents, isotonicity agents, controlled release agents and/or analogues thereof.

### [EXAMPLE]

### 1. Cell Chemotaxis

Boyden chamber assay was used to evaluate the migration of LMVECs. LMVECs were cultured on HuMedia-EB2 with 2% FCS for 8 hours. Cells were seeded on a polycarbonate membrane (Sigma-Aldrich) coated with 10µg/ml of fibronectin by a density of 1.2 × 10⁵cell/cm². 10 ng/ml of CXCL8, CXCL6, CXCL1, CXCL5, and CXCL8-IP10 or IL8-F17LIP10 was added to the bottom of Boyden chamber, respectively. LMVECs were cultured on Boyden chamber at 37°C for 4 hours, and then fixed and stain with Diff-Quick (Harleco). The number of migrated cells was counted by HPFs (X200).

The result represent that a large amount of cell was migrated when present of chemokine CXCL8 (Fig.3).

In Fig. 4, black bar represents chemokine induction, gray bar represents treat with chemokine and CXCL8-IP10 together, and white bar represents treat with chemokine and IL8-17LIP10 simultaneously. According to Fig. 4, CXCL8-IP10 and IL8-17LIP10 both had the effect to inhibit the cell migration, and the IL8-17LIP10 has a better inhibiting effect on cell migration than CXCL8-IP10.

### 2. The anti-tumor effect in BALB/c Nude male mice bearing xenograft tumor via administrated modified chemokine peptides

BALB/c Nude male mice (Bltw:NU-*Foxn1^{nu}*, 4-6 weeks-old) were obtained and maintained in a laminar airflow cabinet under the specific pathogen free conditions. The animals freely accessed to tap water and standard pellet food, and their health was monitored daily. For the nude mouse xenograft assay, the monolayer-cultured GFP-positive PC3 cells (PC-3-GFP) were harvested and inoculated subcutaneously into the right flank of three nude mice with 5 × 10⁶ cell per mouse. After 2 to 4 weeks, tumor was harvested for implantation. At the end of the experiments, the tumor xenografts from these three mice were reset, sliced (1 mm³ sections), and then implanted to prostate tissues of the recipient nude mice under local anesthesia and sterile surgical conditions. A total of 24 animals received implants. Five days later (day 0), the animals were classified into two groups (12 animals/group) for subcutaneous injection of 100 µl of normal saline (control group) or the sequence of the present invention (SEQ ID NO: 12 (IL8-17LIP10), 0.5 mg/kg) (experimental group) for 24 days. GFP fluorescence images of the growing tumors were captured on day 12, 18, and 24 using a digital camera under the optical configuration of a dissection microscope with 515 nm emission filter. The tumor volumes were calculated using a formula: Volume = (length × width²)/2. On day 24, all of mice were sacrificed and GFP fluorescence images of the tumors were captured. Vascular microvessel density was calculated using the formula: Density = microvessel length/tumor area. Tumor samples from each mouse were fixed in 4% paraformaldehyde and embedded in paraffin using standard procedures for subsequent immunohistochemical analyses.

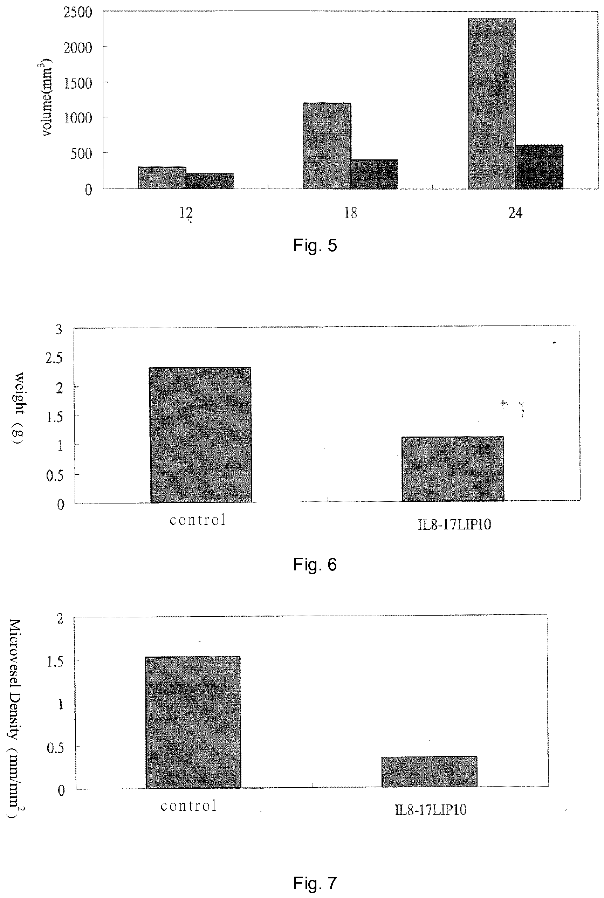

The result shows that the tumor volume of treatment (experimental) group and placebo (control) group on day 12, 18, and 24 (Fig.5). According to Fig.5, the sequence (IL8-17LIP10) of the present invention effectively inhibits tumor growth. The tumor volume of treatment group was reduced by more than 5 times compared with placebo group. This apparent inhibition of tumor volume for treatment group compared with placebo group was sustained and became more pronounced over time.

The result shows that the tumor weight of treatment (experimental) group and placebo (control) group on day 24 (Fig.6). According to Fig. 6, the sequence (IL8-17LIP10) of the present invention effectively inhibits tumor growth, and the tumor weight was decreased by more than 2 times.

### 3. Immunohistochemical Analysis for xenograft tumor tissue

Paraffin-embedded prostate cancer xenograft sections were dewaxed and rehydrated into PBS. In detail, the sections were rinsed three times with PBS and heat-treated for 15 min in 10 mM sodium citrate (pH 6.0). The endogenous peroxidase activities were blocked by treatment with 3% hydrogen peroxide for 10 min. The sections were rinsed three times with PBS again, incubated with a protein-blocking solution (5% normal horse serum in PBS, pH 7.5) for 15 min at room temperature, washed with three times with PBS, and then incubated with a mouse monoclonal anti-VEGF antibody (1 : 50), a rabbit polyclonal anti-NF-κB antibody, or a goat polyclonal anti-CD31 antibody (1 : 50) for 20 hours at 4 °C. After reaction, the sections were washed three times with PBS and incubated with the appropriate dilution of the secondary antibody for 40 min at 37°C. After washing three times with PBS, the sections were incubated with biotinylated goat anti-mouse or anti-rabbit anti-goat poly- immunoglobulin for 30 min in the dark. For color development, the sections were washed with PBS three times, incubated in diaminobenzidine solution for 10 min, and then counterstained with hematoxylin for 1 min (Kollmar et al, 2007). The negative control sections were incubated with PBS instead of the primary antibody. The intensity of the stained sections were measured after converting photographed sections into gray scale (scale 0-255) and represented by integrated optical density (IOD), which was calculated using Image-Pro 6.0 Microsoft. For each group, five mouse tumor xenografts were analyzed. Five photographed sections per tumor, which were chosen randomly in different sections, were used to average gray values of every tumor xenograft (Csillik et al., 2005). Immunohistochemical analyses of CD31 were used to determine microvessel density, and the measurement of microvessel density represents the mean percentage of microvessel area, which was calculated using Image-Pro 6.0 Microsoft.

The result shows that the microvessel density of placebo (control) group and treatment (experimental) group (Fig. 7). According to Fig. 7, the sequence (IL8-17LIP10) of the present invention effectively inhibits angiogenesis on prostate cancer xenograft nude mice.

### 4. The anti-tumor effect in C57BL/6 nudemice bearing xenografttumor via administrated modified chemokine peptides

C57BL/6 nude mice were maintained in a laminar airflow cabinet under specific pathogen. The animals freely accessed to tap water and standard pellet food, and their health was monitored daily. 5 × 10⁶ cells of LLW2 cells (Lewis lung carcinoma) were injected to right quadrant of three mice with 5 × 10⁶ cells per mouse. After 2 to 4 weeks, tumor was harvested for implantation. 24 mice were implanted in this example. After 5 days of implantation, 24 mice were classified into four groups (six mice per group). Group A: four times weekly administration of 500 µg/kg of CXCL8-IP10. Group B: twice weekly administration of 500 µg/kg of CXCL8-IP10. Group C: twice weekly administration of 250 µg/kg of CXCL8-IP10. Group D: 100 µl of saline was subcutaneously administrated every day. The tumor volumes were calculated using a formula: Volume = (length × width²)/2. On day 24, all of mice were sacrificed and GFP fluorescence images of the tumors were captured. Vascular microvessel density was calculated using the formula: Density = microvessel length/tumor area. Tumor samples from each mouse were fixed in 4% paraformaldehyde and embedded in paraffin using standard procedures for subsequent immunohistochemical analyses.

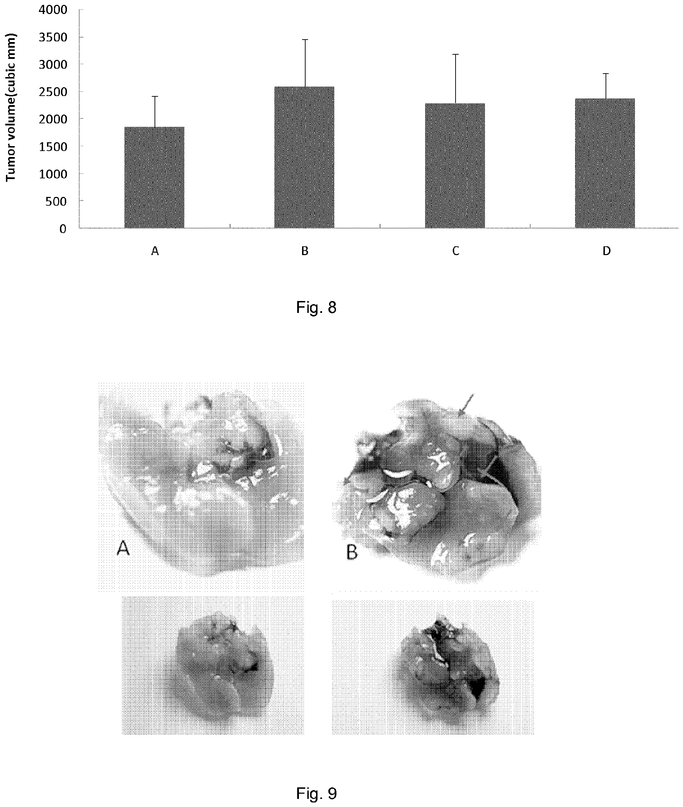

According to Figure 8, Group A shows the best improvement in tumor growth inhibition. On day 24, all of mice were sacrificed to calculate the tumor volume. Compared to Group D, the tumor volume, which were treated with CXCL8-IP10 (500ug/kg) for four times per week, was reduced by 30% comparative to Group A. The results indicate that CXCL8-IP10 had significant inhibition of tumor growth.

According to Fig.9, tumor metastasis was significantly suppressed by CXCL8-IP10. Mice were sacrificed on day 24 to obtain the lung after administration with 500 µg/kg of CXCL8-IP10 four times weekly (Figure 9A) or saline (Figure 9B). The white part indicated by arrow was nidus of the cancer which has spread into lung. After administration of CXCL8-IP10, no metastatic nidus occurred in mice lung.

### 5. Neutrophil Chemotaxis Assay

The neutrophil chemotaxis was evaluated by an improved Boyden chamber microchemotaxis assay. Leucocytes were collected from peripheral blood by a general concentration gradient. Neutrophils were obtained from the bottom of low concentration gradient area, and erythrocyte contamination was removed by hypotonic lysis. The purified neutrophils (5 × 10⁶/ml) were suspended in HBSS solution (400 mg/L KCl, 60 mg/L KH₂PO₄, 8000 mg/L, NaCl, 350 mg/L NaHCO₃, 90 mg/LNaH₂PO₄·7H₂O, and1000 mg/L glucose), and then cultured in Calcein AM (Invitrogen, Stockholm, Sweden) medium at 37°C for 30 minutes. Chemokine (e.g., 20 ng/ml of CXCL8) was placed on the lower chamber alone or in combination with other antagonists (e.g., IL8-IP10F17L), and the purified neutrophils were placed on the upper chamber. An 5 µm pore-size polycarbonate filter was used to separate the upper and lower chamber. After the cells were cultured in a 5% CO₂ humidified atmosphere at 37°C for the30 minutes, the unmigrated cells and filter were removed and the migrated cells were lysed and analyzed using VICTOR3 (Perkin-Elmer, UK, excitation: 485nm; emission: 530nm). A percentage of the cell migration was indicated by chemotaxis index (CI) value.CI = (intensity antagonist-intensity HBSS)/(intensity CXCL8 - intensity HBSS) × 100%, wherein "intensity antagonist" is the cell migration caused by antagonists, "intensity CXCL8" is the cell migration caused by CXCL8, and "intensity HBSS" is the cell migration caused by gravity.

### 6. Expression Level of CXCR1/2 and CXCL8 Genes in Tumor Cells

The RNA of cancer cell lines (Table 1) was extracted by TRIzol reagent (Invitrogen, America), respectively using the standard procedures, and the RNA was quantified by NanoDrop^{®}ND-1000 spectrophotometer. A cDNA reverse-transcription kit (Prime Script^{™} RT reagent kit, Takara, Japan) was used to carry out the reverse transcription of the samples's RNA, and the samples were placed on ice before gene expression analysis. GAPDH was internal control group. RT-PCR reaction was carried out by SYBR (Premix Ex Taq^{™}, Takara, Japan), wherein the primers of CXCL included: 5'-gagcactccataaggcacaaa-3' (forward) and 5'-atggttccttccggtggt-3' (reverse) for CXCL8;5'-gaccaacatcgcagacacat-3' (forward) and 5'-tgcttgtctcgttccacttg-3' (reverse) for CXCR1; 5'-ggctaagcaaaatgtgatatgtacc-3' (forward) and 5'-caaggttcgtccgtgttgta-3' (reverse) for CXCR2.

The gene expression was calculated by the following formula: gene expression = 2^{-ΔΔCt}

**Table 1. Tumor cell line**

| | |
|---|---|
| HCC827 | lung adenocarcinoma |
| HCC827GR | HCC827 gefitinib-resistant |
| H1975 | lung adenocarcinoma |
| H2170 | lung squamous cell carcinoma |
| H157 | oral squamous cell carcinoma |
| CT26 | colon carcinoma cell |
| CL1-0 | lung adenocarcinoma |
| CL1-5 | lung adenocarcinoma |
| PC9 | lung adenocarcinoma |
| H3255 | Non small cell lung carcinoma |
| A549 | lung adenocarcinoma |
| H520 | lung squamous cell carcinoma |
| H460 | Non small cell lung carcinoma |

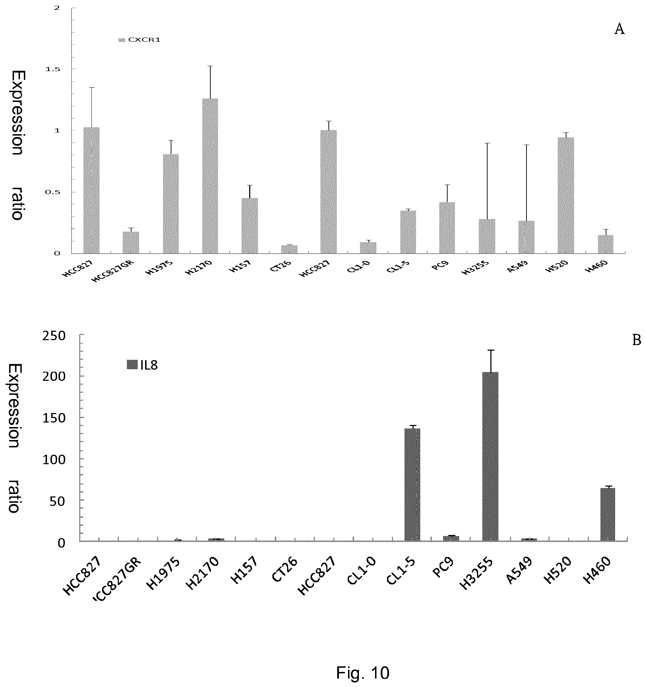

The Fig. 10 illustrates high CXCR1 expression in tumor cells partially by RT-PCR. Many tumor cells highly expressed CXCL8, which is a substance of CXCR1 and CXCR2 receptors. Thus, CXCR1/2 antagonist could be used to inhibit tumor growth and metastasis.

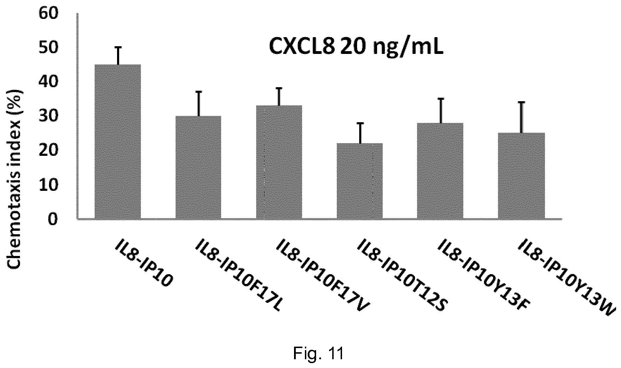

The Fig. 11 illustrates more design of antagonist. The amino acid residue(s) at the first modified position, and/or the second modified position, and/or the third modified position of CXCL8-IP10 peptide was changed to improve the antagonist properties induced by CXCR1/2. These antagonists effectively suppressed the expression of CXCR1/2 receptors, or inhibited tumor growth, drug resistance, metastasis, and angiogenesis associated with high cytokines expression (CXCL1, 2, 3, 5, 6, 7, and 8, etc.) in tumor cells.

As mentioned above, the modified chemokine of the present invention can effectively inhibit tumor growth and angiogenesis and treat cancer.

## Claims

1. A modified chemokine peptide comprising a peptide sequence, wherein the N-terminus of the peptide sequence comprises:
(a) a "Glutamate (E)-Leucine (L)-Arginine (R)" sequence, defined as an A characteristic sequence, wherein the A characteristic sequence is situated at the N-terminus of the modified chemokine peptide;
(b) a "Proline (P)-Alanine (A)-Serine (S)-Glutamine (Q)-Phenylalanine (F)" sequence, defined as a B characteristic sequence, wherein the B characteristic sequence is neighbored to the upstream of the third cysteine (C) counted from N-terminus of a chemokine peptide, and
wherein the modified chemokine peptide comprises the A characteristic sequence, the B characteristic sequence, and a modified position, wherein the modified position is situated at the 17^{th}, 12^{th}, or 13^{th} position counted from the N-terminus of the modified chemokine peptide,
wherein the phenylalanine (F) at the 17^{th} position from the N-terminus of the modified chemokine peptide is substituted with leucine (L), valine (V), or isoleucine (I), or
the threonine (T) at the 12^{th} position from the N-terminus of the modified chemokine peptide is substituted with serine (S),or
the tyrosine (Y) at the 13^{th} position from the N-terminus of the modified chemokine peptide is substituted with leucine (L), phenylalanine (F), Tryptophan (W), or isoleucine (I), and
wherein the source chemokine peptide is selected from the group consisting of SEQ ID NO: 1, and SEQ ID NO: 2.

2. The modified chemokine peptide according to claim 1, wherein the modified chemokine peptide is selected from SEQ ID NO: 10, and SEQ ID NO: 12 (IL8-17LIP10).

3. The modified chemokine peptide according to claim 1, wherein the source protein is SEQ ID NO: 1 and the threonine (T) at the 12^{th} position from the N-terminus of the modified chemokine peptide is substituted with serine (S).

4. The modified chemokine peptide according to claim 1, wherein the source protein is SEQ ID NO: 1 and the tyrosine (Y) at the 13^{th} position from the N-terminus of the modified chemokine peptide is substituted with phenylalanine (F), or Tryptophan (W).

5. A pharmaceutical composition comprising a modified chemokine peptide of any one of claim 1 to claim 4 and a pharmaceutically acceptable excipient.

6. The pharmaceutical composition of claim 5, wherein the modified chemokine peptide is suitable to treat cancer or inhibit tumor growth.

7. A pharmaceutical composition for use in a method of treating a cancer and/or inhibiting tumor growth comprising a therapeutically effective amount of a modified chemokine peptide of any one of claim 1 to claim 4, and a pharmaceutically acceptable excipient.

8. The pharmaceutical composition for use according to claim 7, wherein the cancer comprises prostate cancer, breast cancer, uterine cancer, leukemia, ovarian cancer, endometrial cancer, cervical cancer, colorectal cancer, testicular cancer, lymphoma, rhabdomyosarcoma, neuroblastoma, pancreatic cancer, lung cancer, brain tumor, skin cancer, stomach cancer, oral cancer, liver cancer, laryngeal cancer, gallbladder cancer, thyroid cancer, liver cancer, kidney cancer, or nasopharyngeal carcinoma.

9. The pharmaceutical composition for use according to claim 8, wherein the cancer is **characterized by** cells with a CXCR1 / 2 expression or a high amount of chemokines consisting of CXCL8, CXCL1, CXCL2, CXCL3, CXCL5, CXCL6 and CXCL7, wherein the cancer is **characterized by** cancer cells expressed with chemokine receptors.

## Patentansprüche

1. Modifiziertes Chemokin-Peptid, umfassend eine Peptidsequenz, wobei der N-Terminus der Peptidsequenz umfasst:
(a) eine "Glutamat-(E)-Leucin-(L)-Arginin-(R)"-Sequenz, die als eine charakteristische A-Sequenz definiert ist, wobei sich die charakteristische A-Sequenz am N-Terminus des modifizierten Chemokin-Peptids befindet;
(b) eine "Prolin-(P)-Alanin-(A)-Serin-(S)-Glutamin-(Q)-Phenylalanin-(F)"-Sequenz, die als eine charakteristische B-Sequenz definiert ist, wobei die charakteristische B-Sequenz stromaufwärts des dritten Cysteins (C), gezählt vom N-Terminus eines Chemokin-Peptids, benachbart ist, und
wobei das modifizierte Chemokin-Peptid die charakteristische Sequenz A, die charakteristische Sequenz B und eine modifizierte Position umfasst, wobei die modifizierte Position an der 17., 12. oder 13. Position, gezählt vom N-Terminus des modifizierten Chemokin-Peptids, liegt,
wobei das Phenylalanin (F) an der 17. Position vom N-Terminus des modifizierten Chemokin-Peptids durch Leucin (L), Valin (V) oder Isoleucin (I) substituiert ist, oder das Threonin (T) an der 12. Position vom N-Terminus des modifizierten Chemokin-Peptids durch Serin (S) substituiert ist, oder
das Tyrosin (Y) an der 13. Position vom N-Terminus des modifizierten Chemokin-Peptids durch Leucin (L), Phenylalanin (F), Tryptophan (W) oder Isoleucin (I) substituiert ist, und
wobei das Ausgangs-Chemokin-Peptid ausgewählt ist aus der Gruppe bestehend aus SEQ ID NO: 1 und SEQ ID NO: 2.

2. Modifiziertes Chemokin-Peptid nach Anspruch 1, wobei das modifizierte Chemokin-Peptid aus SEQ ID NO: 10 und SEQ ID NO: 12 (IL8-17LIP10) ausgewählt ist.

3. Modifiziertes Chemokin-Peptid nach Anspruch 1, wobei das Ausgangsprotein SEQ ID NO: 1 ist und das Threonin (T) an der 12. Position vom N-Terminus des modifizierten Chemokin-Peptids durch Serin (S) substituiert ist.

4. Modifiziertes Chemokin-Peptid nach Anspruch 1, wobei das Ausgangsprotein SEQ ID NO: 1 ist und das Tyrosin (Y) an der 13. Position vom N-Terminus des modifizierten Chemokin-Peptids durch Phenylalanin (F) oder Tryptophan (W) substituiert ist.

5. Pharmazeutische Zusammensetzung, umfassend ein modifiziertes Chemokin-Peptid nach einem der Ansprüche 1 bis 4 und einen pharmazeutisch akzeptablen Hilfsstoff.

6. Pharmazeutische Zusammensetzung nach Anspruch 5, wobei das modifizierte Chemokin-Peptid geeignet ist, Krebs zu behandeln oder das Tumorwachstum zu hemmen.

7. Pharmazeutische Zusammensetzung zur Verwendung bei einem Verfahren zur Behandlung von Krebs und/oder zur Hemmung des Tumorwachstums, umfassend eine therapeutisch wirksame Menge eines modifizierten Chemokin-Peptids nach einem der Ansprüche 1 bis 4 und einen pharmazeutisch akzeptablen Hilfsstoff.

8. Pharmazeutische Zusammensetzung zur Verwendung nach Anspruch 7, wobei der Krebs Prostatakrebs, Brustkrebs, Gebärmutterkrebs, Leukämie, Eierstockkrebs, Endometriumkrebs, Gebärmutterhalskrebs, kolorektalen Krebs, Hodenkrebs, Lymphom, Rhabdomyosarkom, Neuroblastom, Bauchspeicheldrüsenkrebs, Lungenkrebs, Gehirntumor, Hautkrebs, Magenkrebs, Mundkrebs, Leberkrebs, Kehlkopfkrebs, Gallenblasenkrebs, Schilddrüsenkrebs, Leberkrebs, Nierenkrebs oder Nasopharynxkarzinom umfasst.

9. Pharmazeutische Zusammensetzung zur Verwendung nach Anspruch 8, wobei der Krebs durch Zellen mit einer CXCR1/2-Expression oder einer hohen Menge an Chemokinen, bestehend aus CXCL8, CXCL1, CXCL2, CXCL3, CXCL5, CXCL6 und CXCL7, gekennzeichnet ist, wobei der Krebs durch Krebszellen, die mit Chemokinrezeptoren exprimiert werden, gekennzeichnet ist.

## Revendications

1. Peptide de chimiokine modifiée comprenant une séquence peptidique, dans lequel l'extrémité N-terminale de la séquence peptidique comprend :
(a) une séquence « glutamate (E)-leucine (L)-arginine (R) », définie comme une séquence caractéristique A, la séquence caractéristique A étant située à l'extrémité N-terminale du peptide de chimiokine modifiée ;
(b) une séquence « proline (P)-Alanine (A)-sérine (S)-glutamine (Q)-phénylalanine (F) », définie comme une séquence caractéristique B, la séquence caractéristique B étant voisine de l'amont de la troisième cystéine (C) comptée à partir de l'extrémité N-terminale d'un peptide de chimiokine, et
dans lequel le peptide de chimiokine modifiée comprend la séquence caractéristique A, la séquence caractéristique B et une position modifiée, la position modifiée étant située à la 17^{ème}, 12^{ème} ou 13^{ème} position comptée à partir de l'extrémité N-terminale du peptide de chimiokine modifiée,
dans lequel la phénylalanine (F) à la 17^{ème} position de l'extrémité N-terminale du peptide de chimiokine modifiée est substituée avec une leucine (L), une valine (V) ou une isoleucine (I), ou
la thréonine (T) à la 12^{ème} position de l'extrémité N-terminale du peptide de chimiokine modifiée est substituée avec une sérine (S), ou
la tyrosine (Y) à la 13^{ème} position de l'extrémité N-terminale du peptide de chimiokine modifiée est substituée avec une leucine (L), une phénylalanine (F), un tryptophane (W) ou une isoleucine (I) et
dans lequel le peptide de chimiokine source est choisi dans le groupe constitué par SEQ ID NO: 1 et SEQ ID NO: 2.

2. Peptide de chimiokine modifiée selon la revendication 1, dans lequel le peptide de chimiokine modifiée est choisi parmi SEQ ID NO: 10 et SEQ ID NO: 12 (IL8-17LIP10).

3. Peptide de chimiokine modifiée selon la revendication 1, dans lequel la protéine source est SEQ ID NO: 1 et la thréonine (T) à la 12ème position de l'extrémité N-terminale du peptide de chimiokine modifiée est substitué avec une sérine (S).

4. Peptide de chimiokine modifiée selon la revendication 1, dans lequel la protéine source est SEQ ID NO: 1 et la tyrosine (Y) à la 13^{ème} position de l'extrémité N-terminale du peptide de chimiokine modifiée est substituée avec une phénylalanine (F) ou un tryptophane (W).

5. Composition pharmaceutique comprenant un peptide de chimiokine modifiée selon l'une quelconque de la revendication 1 à 4 et un excipient pharmaceutiquement acceptable.

6. Composition pharmaceutique selon la revendication 5, dans laquelle le peptide de chimiokine modifiée est approprié pour traiter le cancer ou inhiber la croissance tumorale.

7. Composition pharmaceutique destinée à être utilisée dans un procédé de traitement d'un cancer et/ou d'inhibition de la croissance tumorale comprenant une quantité thérapeutiquement efficace d'un peptide de chimiokine modifiée selon l'une quelconque de la revendication 1 à 4 et un excipient pharmaceutiquement acceptable.

8. Composition pharmaceutique destinée à être utilisée selon la revendication 7, dans laquelle le cancer comprend le cancer de la prostate, le cancer du sein, le cancer de l'utérus, la leucémie, le cancer des ovaires, le cancer endométrial, le cancer cervical, le cancer colorectal, le cancer testiculaire, le lymphome, le rhabdomyosarcome, le neuroblastome, le cancer pancréatique, le cancer du poumon, le cancer du cerveau, le cancer de la peau, le cancer de l'estomac, le cancer oral, le cancer du foie, le cancer laryngien, le cancer de la vésicule biliaire, le cancer de la thyroïde, le cancer du foie, le cancer du rein ou le carcinome nasopharyngien.

9. Composition pharmaceutique destinée à être utilisée selon la revendication 8, dans laquelle le cancer est **caractérisé par** des cellules avec une expression de CXCR 1 / 2 ou une quantité élevée de chimiokines constituées de CXCL8, CXCL1, CXCL2, CXCL3, CXCL5, CXCL6 et CXCL7, le cancer étant **caractérisé par** des cellules cancéreuses exprimées avec des récepteurs de chimiokine.
